(19) Europäisches Patentamt
European Patent Office
Office européen des brevets

(11) EP 1 477 566 B1

(12) **EUROPEAN PATENT SPECIFICATION**

(45) Date of publication and mention
of the grant of the patent:
**26.09.2007 Bulletin 2007/39**

(21) Application number: **03734900.8**

(22) Date of filing: **31.01.2003**

(51) Int Cl.:
*C12P 21/00* (2006.01)   *C12P 21/02* (2006.01)

(86) International application number:
**PCT/JP2003/000995**

(87) International publication number:
**WO 2003/064671 (07.08.2003 Gazette 2003/32)**

(54) **GERM EXTRACT FOR CELL-FREE PROTEIN SYNTHESIS AND PROCESS FOR PRODUCING THE SAME**

KEIMEXTRAKT ZUR ZELLFREIEN PROTEINSYNTHESE UND VERFAHREN ZU DESSEN HERSTELLUNG

EXTRAIT DE GERMES PERMETTANT LA SYNTHESE ACELLULAIRE DE PROTEINES ET PROCEDE PERMETTANT DE PRODUIRE CES DERNIERES

(84) Designated Contracting States:
**AT BE BG CH CY CZ DE DK EE ES FI FR GB GR HU IE IT LI LU MC NL PT SE SI SK TR**

(30) Priority: **31.01.2002 JP 2002023138**
**31.01.2002 JP 2002023139**
**31.01.2002 JP 2002023140**

(43) Date of publication of application:
**17.11.2004 Bulletin 2004/47**

(73) Proprietor: **CellFree Sciences Co., Ltd.**
**Yokohama-shi, Kanagawa 230-0046 (JP)**

(72) Inventors:
• **ENDO, Yaeta**
**Matsuyama-shi, Ehime 791-8016 (JP)**
• **Dohi, Naoki,**
**c/o Mitsubishi Chemical Corporation**
**Kurashiki-shi, Okayama 712-8054 (JP)**
• **Nakagawa, Makoto,**
**c/o Mitsubishi Chemical Corp.**
**Kurashiki-shi, Okayama 712-8054 (JP)**

(74) Representative: **De Clercq, Ann G. Y. et al**
**De Clercq, Brants & Partners c.v.**
**Edgard Gevaertdreef 10a**
**9830 Sint-Martens-Latem (BE)**

(56) References cited:
EP-A- 1 479 776          EP-A- 1 489 188
WO-A-02/095377          WO-A1-00/68412
WO-A1-93/07287          CN-A- 1 288 655
JP-A- 6 225 783          JP-A- 2000 236 896
US-A- 5 593 856

• MADIN KAIRAT ET AL: "A highly efficient and robust cell-free protein synthesis system prepared from wheat embryos: Plants apparently contain a suicide system directed at ribosomes" PROCEEDINGS OF THE NATIONAL ACADEMY OF SCIENCES OF USA, NATIONAL ACADEMY OF SCIENCE. WASHINGTON, US, vol. 97, no. 2, 18 January 2000 (2000-01-18), pages 559-564, XP002286586 ISSN: 0027-8424
• DATABASE BIOSIS BIOSCIENCES INFORMATION SERVICE, PHILADELPHIA, PA, US; 1994, BARNABAS J ET AL: "Time saving method for protoplast isolation, transformation and transient gene expression assay in barley" Database accession no. PREV199497397961
• DENYER K ET AL: "The contributions of adenosine 5'-diphosphoglucose pyrophosphorylase and starch-branching enzyme to the control of starch synthesis in developing pea embryos" PLANTA, vol. 197, no. 1, 1995, pages 57-62, XP008066854 ISSN: 0032-0935

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

**Description**

**TECHNICAL FIELD**

The present invention relates to embryo extract for cell-free protein synthesis and to a method for preparing the same. More specifically, the invention relates to embryo extract for cell-free protein synthesis having high synthesis efficiency and to a method of preparing the same in an industrially efficient manner.

**BACKGROUND ART**

Intracellular protein synthesis reactions proceed through the steps of first transcribing genetic information from DNA that bears the information into mRNA, whereafter a ribosome translates the information from this mRNA to synthesize a protein. Currently, in terms of methods for performing protein synthesis, which normally occurs in the cell, *ex vivo*, such as in a test tube, a great deal of research is underway into cell-free protein synthesis wherein, for example, ribosomes are extracted from an organism and these are used to perform protein synthesis reactions *in vitro* (JP-06-098790-A, JP-06-225783-A, JP-07-000194-A, JP-09-000291-A, JP-07-147992-A). *Escherichia coli,* plant embryo, rabbit reticulocytes, and the like are used as sources of ribosomes for these methods.

Methods for obtaining embryo extract containing ribosomes from plant embryo for use in cell-free protein synthesis commonly consist of grinding plant seeds, whereafter the seed coat and the endosperm fractions are removed to produce a crude embryo fraction, which is further washed so as to eliminate the endosperm component, whereafter this is ground, extracted and purified.

In conventional methods for pulverizing the embryo, it was thought necessary to grind the embryo as finely as possible so as to more efficiently extract soluble components comprising protein synthesis components such as ribosomes and tRNA. However, as the embryos are small and strong, they cannot easily be ground. Accordingly, in conventional methods it was common to freeze the washed embryo in liquid nitrogen or the like and then pulverize the embryo by milling or crushing with a mortar, stamp mill, bowl mill, or the like. An extraction solvent was then added to the embryo which had been pulverized in this manner, this was stirred, and thereafter a solution containing embryo extraction product (embryo extract) was recovered and purified by centrifuging or the like, for use as an "enzyme stock solution" for cell-free protein synthesis reactions. This "enzyme stock solution" contains numerous unnecessary components that are not involved in protein synthesis reactions and may also contain substances that have an inhibitory effect on protein synthesis.

Furthermore, the extract produced by the conventional methods described above did not contain a sufficient quantity of tRNA for the protein synthesis reactions, which necessitated the addition of separately prepared tRNA. Furthermore, it was difficult to produce large quantities of extract in a short period of time with the conventional methods described above.

WO 00/68412 discloses that embryos obtained from plant seeds may contain endosperm components and that the latter can be substantially eliminated by treating the embryos with a surfactant, preferably in combination with ultrasonication. The so treated embryos of WO 00/68412 are subsequently pulverised using a mortar to prepare an extract.

WO 93/07287 discloses a system for coupling transcription and translation from DNA using solutions comprising a eukaryotic cell-free extracts.

CN-A-1 288 655 discloses an assay method for storage-property rice, which uses a cut rice seed embryo.

Barnabas et al. 1994 (Agricultural sciences in Finland 3: 199-205) discloses a method for isolation of protoplasts from barley using enzymatic digestion of chopped embryo and scutellum parts.

Denyer et al. 1995 (Planta 197: 57-62) describes isolation of plastids from pea embryos to determine the activities of ADP-glucose pyrophosphorylase and starch-branching enzyme therein. Although the plastid isolation involves chopping of the embryos in an extraction medium, Denyer et al. 1995 subsequently only recover the pellet which contains the plastids. Denyer et al. do not at all relate to cell-free protein synthesis.

**DISCLOSURE OF THE INVENTION**

In order to solve the problems described above, the present inventors studied methods for increasing the protein synthesis efficiency of embryo extracts used in cell-free protein synthesis systems, for manufacturing this extract at industrially practicable levels of efficiency, and for extracting from plant embryo the factors necessary for efficient protein synthesis. In this manner, the present invention was achieved.

That is to say, the present invention is as described hereinafter.

(1) A cell-free protein synthesis method comprising:

- preparing plant embryo extract comprising the steps of:

(a)

(1) mincing a plant embryo by chopping, and
(2) extracting the minced plant embryo with an extracting solvent,

or

(a)' mincing a plant embryo in the presence of extracting solvent by chopping and extracting the plant embryo which has been minced, wherein said steps of mincing and extracting are carried out simultaneously and,

-    using the plant embryo extract in cell-free protein synthesis.

(2) The cell-free protein synthesis method set forth above in (1), wherein the mincing of a plant embryo by chopping is by means of a high speed rotary blade.

(3) The cell-free protein synthesis method set forth above in any of (1) or (2), wherein the step of mincing the plant embryo is in the presence of extracting solvent.

(4) The cell-free protein synthesis method set forth above in any of (1) to (3), wherein the extracting solvent comprises at least one substance selected from the group of: a buffer solution, potassium ions, magnesium ions, and a thiol antioxidant.

(5) The cell-free protein synthesis method set forth above in any of (1) to (4), wherein the plant embryo is the embryo of wheat, barley, rice or corn.

(6) The cell-free protein synthesis method set forth above in any of (1) to (5), wherein the plant embryo is substantially uncontaminated by an endosperm component.

(7) A cell-free protein synthesis solution characterised by comprising :

-    an embryo extract produced by a preparation method comprising the steps of:

(a)

(1) mincing a plant embryo by chopping, and
(2) extracting the minced plant embryo with an extracting solvent,

or

(a') mincing a plant embryo in the presence of extracting solvent by chopping and extracting the plant embryo which has been minced, wherein said steps of mincing and extracting are carried out simultaneously,

wherein the plant embryo is substantially uncontaminated by an endosperm component, and,

-    and ATP, GTP, creatine phosphate creatine kinase, L-amino acids, potassium ions and magnesium ions.

(8) The cell-free protein synthesis solution set forth above in (7), wherein the mincing of the plant embryo by chopping is by means of a high speed rotary blade.

(9) The cell free protein synthesis solution set forth above in any of (7) or (8), wherein the step of mincing the plant embryo is in the presence of extracting solvent.

(10) The cell-free protein synthesis solution set forth above in any of (7) to (9), wherein the extracting solvent comprises at least one substance selected from the group of: a buffer solution, potassium ions, magnesium ions, and a thiol antioxidant.

(11) The cell-free protein synthesis solution seth forth above in any of (7) to (10), wherein the plant embryo is the embryo of wheat, barley, rice or corn.

(12) The cell-free protein synthesis solution set forth above in any of (7) to (11), said cell-free protein synthesis solution having sufficient protein synthesis activity without tRNA being added.

(13) A kit for performing cell-free protein synthesis characterised by comprising the cell-free protein synthesis solution set forth above in any of (7 or (12).

**BRIEF DESCRIPTION OF THE DRAWINGS**

Figure 1 is a graph illustrating the particle size distribution of ground embryo produced in Example 1.

Figure 2 is a graph illustrating the particle size distribution of ground embryo produced in Comparative Example 1.

Figure 3 is a graph illustrating the fluorescent intensity of GFP synthesized in Example 2 and Comparative Example 2.

Figure 4 is a graph illustrating the results for synthesis of DHFR in Example 7 and Comparative Example 6. The vertical axis shows radioactivity incorporated into the protein (amount of $[^{14}C]$-leucine incorporated: dpm/5 $\mu$l) and the horizontal axis shows of the amount of tRNA added.

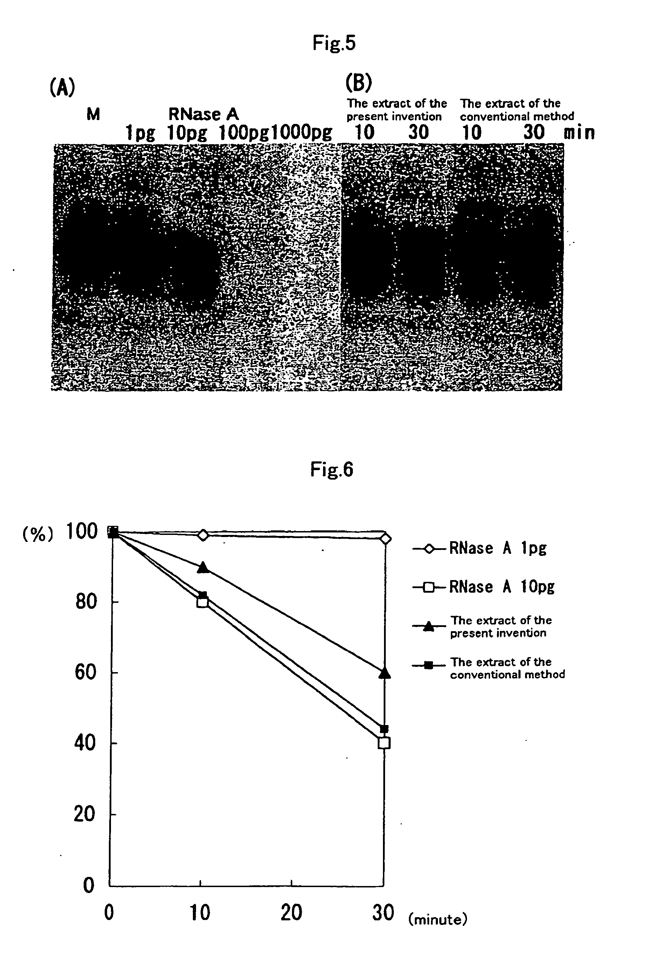

Figure 5 is an electrophoresis profile illustrating the results of measuring the ribonuclease activity of the embryo extract of the present invention and embryo extract produced by extraction according to conventional methods. (A) shows RNA degradation when substrate mRNA is treated with solutions containing RNase A, prepared at four different levels of concentration. (B) shows RNA degradation when substrate mRNA is treated with the embryo extract of the present invention, and separately with embryo extract produced by extraction according to conventional methods. Note that M indicates a marker.

Figure 6 is a graph showing the amount of RNA remaining when the intensities of the bands in the electrophoresis profile obtained in Figure 5 are measured, with no RNase A treatment considered as 100% (no RNA degradation).

**DETAILED DESCRIPTION OF THE INVENTION**

Hereinafter, the present invention is described in further detail.

The plant embryo used in the present invention is obtained from plant seed. Examples of plant seeds that can be used normally include those selected from such plants of the *Gramineae* family as wheat, barley, rice and corn. Among these, preferred plant seeds for use in the present invention include the wheat, barley, rice and corn mentioned above, but wheat is particularly preferred.

As the amount of embryo contained in plant seeds is small, it is desirable to eliminate, to as great an extent as is possible, components other than embryo, so as to recover the embryo efficiently. Normally, mechanical force is first applied to the plant seeds so as to produce a mixture comprising embryo, crushed endosperm and crushed seed coat. The crushed endosperm, crushed seed coat and the like are removed from this mixture, so as to produce a crude embryo fraction (a mixture primarily composed of embryo but also containing crushed endosperm and crushed seed coat). It suffices that the force applied to the plant seed be of a strength sufficient to separate the embryo from the plant seed.

Normally, conventional grinding equipment is used to grind the plant seeds, so as to produce a mixture containing embryo, crushed endosperm and crushed seed coat.

The plant seeds can be ground using commonly known grinding apparatus but it is preferable to use grinding apparatus of the type that applies impact force to the material that is ground, such as a pin mill or a hammer mill. The degree of grinding may be suitably chosen according to the size of the embryo of the plant seed that is used. For example, wheat grain is usually ground to a maximum length of no greater than 4 mm, and is preferably ground to a maximum length of no greater than 2 mm. Furthermore, it is preferable that the grinding be performed by dry grinding.

Next, a crude embryo fraction is recovered from the ground plant seed produced, using classifier well-known *per se,* such as a sieve. For example, in the case of wheat grain, a crude embryo fraction is normally recovered using a mesh size of 0.5 to 2.0 mm, and preferably 0.7 to 1.4 mm. Furthermore, if necessary, the seed coat, endosperm, dust and the like contained in the crude embryo fraction produced can be removed by wind force or electrostatic force.

It is also possible to produce a crude embryo fraction by employing methods that make use of the difference in the specific gravities of embryo, seed coat and endosperm, such as heavy media separation. In order to obtain a crude embryo fraction containing a greater quantity of embryo, a plurality of the methods described above may be combined. Furthermore, it is possible to select the embryo from the crude embryo fraction produced either visually or using a color sorter, or the like.

As the endosperm component may adhere to the embryo fraction produced in this manner, it is preferable that this be washed in order to purify the embryo. It is preferable that this be washed by dispersing/suspending the embryo fraction in cold water or a cold aqueous solution at a temperature that is normally no greater than 10°C and preferably no greater than 4°C and washed until the washing solution is no longer clouded. It is more preferable that the embryo fraction be dispersed/suspended in an aqueous solution containing a surface active agent, which is normally at a temperature of no more than 10°C and preferably at a temperature of no more than 4°C, and washed until the washing solution is no longer clouded. It is preferable that the surface active agent be nonionic, and a wide variety of surface active agents can be used so long as these are nonionic. Specific examples of suitable substances include Brij, Triton, Nonidet P40, Tween, and the like, which are polyoxyethylene derivatives. From among these, Nonidet P40 is the most suitable. These nonionic surface active agents can, for example, be used at a concentration of 0.5%.

The washing treatment may be either one of washing with water or an aqueous solution, or washing with a surface active agent. Alternatively, the two may be used together. Furthermore, this washing may be combined with an ultrasound treatment.

After selecting the plant embryo from the ground product, which was produced by grinding the plant seed as described above, the intact (capable of germinating) embryo produced by washing is minced in the presence, or absence, of an extracting solvent and, in the latter case, this minced plant embryo undergoes extraction with an extracting solvent, so as to produce a embryo extraction product and a solution containing the embryo extraction product (hereinafter, simply, embryo extract) is separated and purified.

In the present invention, the embryo is minced by chopping. It is particularly desirable that chopping be used when the embryo is minced in the absence of an extracting solvent. Herein, the expression "mince by chopping" means breaking down the plant embryo under conditions that minimize, as compared to conventional milling or crushing, wherein such well-known equipment as a mortar and pestle, a stamp mill, a bowl mill or the like can be used to pulp the embryo by abrasion or by pressure, the breakdown of parts of the plant embryo such as cellular membranes, cell walls, and organelles such as mitochondria, chloroplasts and the cell nucleus.

There are no particular restrictions on the apparatus and methods that can be used in mincing the embryo by chopping, so long as the conditions described above are satisfied, but it is preferable that devices having a high-speed rotary blade, such as a Waring blender, be used. The speed of the rotating blade is normally no less than 1,000 rpm and preferably no less than 5,000 rpm, but is normally no greater than 30,000 rpm, and preferably no greater than 25,000 rpm. The running time for the rotating blade is normally no less than five seconds and preferably no less than 10 seconds. There is no particular upper limit on the running time but this is normally no more than 10 minutes and preferably no more than five minutes. There are no particular restrictions on the temperature when the embryo is minced, so long as the temperature does not impair the embryo's capacity for protein synthesis, but this temperature is preferably no greater than 10°C and within a temperature range in which the mincing operation is possible, and on the order of 4°C is particularly preferable.

Examples of particularly suitable methods for mincing the embryo include mincing by chopping as described above. The use of such methods does not completely break down cellular membranes, cell walls or such organelles as the cell nucleus, and the like of the embryo, but leaves at least some part thereof undestroyed. That is to say, as the cellular membranes, cell walls and organelles such as the cell nucleus, and the like, of the embryo are not broken down to a greater degree than is necessary, it is possible to efficiently extract substances necessary to protein synthesis, such as RNA, ribosomes and the like, which are localized within the cytoplasm, at high degrees of purity, without contamination by impurities contained therein, such as lipids and DNA.

The embryo can be minced in the presence of an extracting solvent or in the absence of the extracting solvent.

In cases where the embryo is minced in the presence of an extracting solvent, the procedure can be performed as described in specific detail below.

By mincing the embryo by chopping in the presence of an extracting solvent, the step of mincing the plant embryo and the step of extracting the plant embryo, which has been minced, with an extracting solvent are carried out simultaneously. In this case, the embryo is mixed with an amount of extracting solvent sufficient for extraction, and the embryo is minced in the presence of the extracting solvent. In terms of the amount of extracting solvent used for each gram of unwashed embryo, this is normally no less than 0.1 ml, preferably no less than 0.5 ml, and more preferably no less than 1 ml. There is no particular upper limit on the amount of extracting solvent but this is normally no more than 10 ml, and preferably no more than 5 ml, for each gram of unwashed embryo. The mixture is separated by centrifuging or the like and the supernatant produced is recovered as the embryo extract. This may be subject to a further purification step, using gel filtration or the like.

If the mincing and the solvent extraction are performed at the same time in a single step, the embryo extract can be produced efficiently, and even with limited breakdown of such parts of the embryos as cellular membranes, cell walls and organelles such as the cell nucleus, it is possible to more thoroughly extract factors necessary for protein synthesis, which are localized within the cytoplasm, as compared to extracting after mincing the embryo.

Furthermore, in cases where the embryo is minced in the absence of the extracting solvent, the procedure can be performed as described in specific detail below. First, the extracting solvent is added to embryo which has been minced, preferably by impact or chopping, without excessively breaking down cellular membranes, cell walls or organelles such as the cell nucleus, and after stirring, the mixture is separated by centrifuging or the like, and the supernatant obtained is collected as the embryo extract. This may be subject to a further purification step, using gel filtration or the like. In terms of the amount of extracting solvent to be used and upper limits thereon, figures may be used which are similar to those in the case described above for mincing the embryo in the presence of the extracting solvent.

In terms of the embryo which is to be minced, this may be frozen as was conventional, or an unfrozen embryo may be used, but the use of unfrozen embryo is preferred.

An aqueous solution comprising at least one of: a buffer solution, potassium ions, magnesium ions and a thiol antioxidant, may be used as the extracting solvent. Furthermore, calcium ions and L-amino acids may be added as necessary. For example, solutions comprising N-2-hydroxyethylpiperazine-N'-2'-ethanesulfonic acid (HEPES)-KOH, potassium acetate, magnesium acetate, L-amino acids and/or dithiothreitol and a solution produced by partially modifying the method of Patterson *et al.* (solutions comprising HEPES-KOH, potassium acetate, magnesium acetate, calcium chloride, L-amino acids and/or dithiothreitol) can be used as the extracting solvent. The compositions and concentrations of the various components in the extracting solvent are already known *per se*, and compositions and concentrations commonly used in the preparation of embryo extraction products for cell-free protein synthesis may be adopted.

Gel filtration may, for example, be performed using gel filtration apparatus which has been pre-equilibrated with the solution (a solvent containing HEPES-KOH, potassium acetate, magnesium acetate, dithiothreitol, or L-amino acids). The compositions and concentrations of the various components in the gel filtration solution are already known *per se,* and compositions and concentrations commonly used in the preparation of embryo extracts for cell-free protein synthesis may be adopted.

Following gel filtration, the embryo extract may be contaminated with microorganisms, and in particular, with spores such as those of filamentous bacteria (mold). It is, therefore, preferable that these microorganisms be eradicated. The proliferation of microorganisms is particularly observed in long-term (more than one day) cell-free protein synthesis reactions. It is, therefore, important to prevent this. There are no particular restrictions on the means for eradicating microorganisms, but the use of antimicrobial filters is preferred. There are no particular restrictions on the pore size for the filter, so long as this is a size capable of eradicating microorganisms with which the embryo extract may be contaminated, but 0.1 to 1 $\mu$m is normally suitable and 0.2 to 0.5 $\mu$m is preferred. It is of note that the spore-size of *Bacillus subtilis,* which is in the small class, is 0.5 $\mu$m x 1 $\mu$m and therefore the use of a 0.20 micrometer filter (for example the Minisart™ by Sartorius) is efficient for removing spores. When filtering, it is preferable that a filter having a large pore size be used first, whereafter a filter having a pore size capable of eliminating microorganisms by which the embryo extract may be contaminated is used.

In the present invention, it is preferable that the embryo be minced in the presence of the extracting solvent and it is particularly preferable that the embryo be minced by chopping in the presence of the extracting solvent.

The embryo extract obtained in this manner is purified so as to be substantially uncontaminated by endosperm comprising substances, contained or retained by the source cell itself, which inhibit protein synthesis function (substances that act on mRNA, tRNA, translation factor proteins, ribosomes and the like so as to inhibit the function thereof such as tritin, thionine, ribonuclease, phosphatase and the like). Herein, the expression "substantially uncontaminated by endosperm" refers to embryo extracts from which endosperm components have been removed to an extent that ribosomes are substantially not deadenylated. Furthermore, the expression "ribosomes are substantially not deadenylated" means that the ribosome deadenylation is less than 7%, and preferably 1 % or less.

Furthermore, by implementing a step wherein low molecular weight substances that inhibit protein synthesis function, and by which this embryo extract may be contaminated, are removed by dialysis or the like, it is possible to produce a embryo extract having even better protein synthesis function. Is preferable that this removal step be performed in the presence of stabilizing components such as ATP, GTP, amino acids, or the like.

Moreover, the embryo extract of the present invention, such as the embryo extract obtained by the procedure described above, is characterized by having a reduced ribonuclease activity. Herein, the term "reduced" is intended to mean that, when compared to plant embryo extracts for cell-free protein synthesis produced by conventional well-known methods for preparing embryo extracts, the activity thereof is reduced, and the term specifically refers to embryo extract wherein the ribonuclease activity, as converted to RNase A, is no greater than 85%, preferably no greater than 80% and most preferably no greater than 75% of the ribonuclease activity of embryo extract produced by mincing plant embryo in the absence of an extracting solvent by milling or crushing (which is to say, by conventional methods). For example, a embryo extract having reduced ribonuclease activity of the present invention has a ribonuclease activity of no greater than 10 pg/$\mu$l, as converted to RNase A.

Ribonuclease activity can be measured by methods already known *per se*, but specifically, using mRNA as a substrate for ribonuclease (it will be clear to those skilled in the art that there are no particular restrictions on the type of mRNA, and that the mRNA may be freely chosen so long as the same type of mRNA is used in the comparative studies, which are based on embryo extract [embryo extract produced by conventional extraction methods] that serves as a control for the embryo extract of the present invention) and finding the degree of RNA degradation when this mRNA is treated with the various embryo extracts, it is possible to discover the relative ribonuclease activity contaminating the embryo extract of the present invention. Furthermore, by comparing the degree of degradation by this RNase to that of a ribonuclease of known concentration, which serves as a standard preparation, such as RNase A, it is possible to discover the amount of ribonuclease contaminating the embryo extract of the present invention. In the present specification, "converted to RNase A" means that the value is that would be obtained if RNase A, which is a ribonuclease of a known concentration used as a standard preparation, were used. The specific procedure is described below.

Furthermore, the embryo extract of the present invention is such that, for example, when the optical density at 260 nm (O.D.) ($A_{260}$) is 90, the embryo extract obtained by the procedure described above has a DNA content of no more than 230 μg/ml, preferably no more than 200 μg/ml, and more preferably no more than 180 μg/ml. In the present invention, the DNA content is measured according to the following method.

Method for measuring DNA content

DNA content is measured using a microplate fluorophotometer (SPECTRAmax GEMINI XS, Molecular Devices) using PicoGreen dsDNA quantitation reagent (Molecular Probes), with Calf Thymus DNA Standard (Pharmacia Biotech) as a standard sample.

First, 10 μl of proteinase K (10 mg/ml) are added to 200 μl of sample and this is reacted overnight at 55°C. After extracting the reaction solution in an equal volume of phenol, this is deproteinized by further extraction with an equal volume of phenol/chloroform (1:1) and ethanol precipitated by adding a 1/10 volume of 3 mol/l sodium acetate and 2 volumes of ethanol. After washing with 70% ethanol, this is dissolved in 10 mM/l tris hydrochloride buffer (pH 8.0) (TE) containing 1 mM/l sodium ethylenediaminetetraacetate. RNaseA (10 mg/ml) in the amount of 5 μl are added to the solution and reacted overnight at 37°C, then extracted with phenol and phenol/chloroform as described above to remove the RNA. The sample is ethanol precipitated, washed and dissolved in 200 μl of TE. Picogreen dsDNA quantitation reagent, diluted 200 fold with TE, and the sample, which has been diluted to a suitable concentration with TE, are mixed at a 1:1 ratio, fluorescence is measured at 485/530 nm excitation/emission (fluorescent) wavelengths, and the DNA content is calculated using a standard curve.

Furthermore, the embryo extract of the present invention is such that when the optical density at 260 nm (O.D.) ($A_{260}$) is 90, the total fatty acid content is no more than 0.03 g/100 g, preferably no more than 0.02 g/100 g, and more preferably no more than 0.018 g/100 g. In the present invention, total fatty acid content is a value measured according to the following method (gas chromatography method). In the present invention, total fatty acid content is the total content of palmitic acid, oleic acid, and linoleic acid.

Method for measuring total fatty acid content

Acid hydrolysis was performed by adding 2 mg of heptadecanoic acid (internal standard), 4 ml of ethanol, 6.7 ml of ion exchanged water and 8.3 ml of 12 mol/l hydrochloric acid to 5ml of sample. Next, 16 ml of ethanol were added, extraction was performed by adding 100 ml of a mixture of diethyl ether/petroleum ether (volume ratio 1:1), and this was twice extracted with 60 ml of this mixture. After washing with water to remove the extracting solvent, saponification and methylesterification were performed according to ACOS Official Method Ce 1 b-89 (1997), whereafter measurements were performed by gas chromatography under the following operating conditions.

*Gas chromatography operation's conditions*

Measurement device: Shimadzu GC-17A

Detector: FID

Column: J & W DB-23, φ 0.25 mm x 30 mm, df. 0.25 μm

Column temperature: 70°C (1 minute retention) → 170°C (Temperature rise: 10°C/min.) → 210°C (Temperature rise: 1.2°C/min)

Inlet temperature: 250°C

Detector temperature: 250°C

Gas flow: helium 1.5 ml/minute

Gas pressure: hydrogen 60 kPa, air 50 kPa

Injector: splitless

The contents of the various fatty acids (palmitic acid, oleic acid and linoleic acid) were calculated based on readings obtained according to the following formula, so as to determine the total fatty acid content.

$$\text{Content in each fatty acid (g/100 g)} = (E \times F \times H/D \times G) \times 0.1$$

(In the formula, D represents the peak area for heptadecanoic acid, E represents the peak areas for each of the fatty acids, F represents the amount of heptadecanoic acid added (mg), G represents the amount of sample used, and H represents the sensitivity correction coefficient, which is the previously measured sensitivity of each fatty acid to heptadecanoic acid.)

Furthermore, when the embryo extract of the present invention, such as the embryo extract obtained according to the procedure described above, is used in cell-free protein synthesis, it is possible to limit the consumption of energy sources such as ATP, as compared to the use of embryo extract produced by conventional extraction methods. This suggests that the phosphatase activity of the embryo extract of the present invention may be reduced.

The present invention also provides a cell-free protein synthesis method characterized by using the embryo extract of the present invention.

Other than the use of the embryo extract obtained in the manner described above, the cell-free protein synthesis method of the present invention is performed in the same manner as conventional methods. This method may be a well-known batch method, or a method wherein amino acids and an energy source are continuously supplied, such as the continuous cell-free protein synthesis systems of Spirin *et al.* (A.S. Spirin et al., [1988] Science, 242, 1162-1164) and Yokoyama *et al.* (Kikawa et al., The 21st meeting of Molecular Biology Society of Japan, WID6) As the reaction stops when protein synthesis is performed over a long period of time, using the batch method, the use of the latter systems in which amino acids and an energy source are continuously provided, which allows the reaction to be maintained over a long period of time, make further increases in efficiency possible. Furthermore, if a continuous supply system is used for protein synthesis, dialysis can be used. For example, large-scale continuous preparation of proteins is possible with an ultrafiltration dialysis system using the embryo extract of the present invention as the internal dialysis solution, and a mixture containing an energy source and amino acids as the external dialysis solution. Here, examples of the energy source include adenosine triphosphate (ATP), guanosine triphosphate (GTP), creatine phosphate, and the like, and examples of the amino acids include the 20 types of L-amino acids.

As the embryo extract of the present invention (embryo extract produced by a method characterized in that the embryo is minced in the presence of an extracting solvent, or embryo extract produced by a method characterized in that the embryo is minced in the absence of an extraction solvent by impact or chopping and subsequently extracted with an extracting solvent) is such that cellular membranes, cell walls and such organelles as the cell nucleus of embryo and the like are not broken down more than is necessary during preparation, thus greatly reducing such as contamination by ribonuclease. Thus, as tRNA, which is a requisite for protein synthesis, is subject to little degradation by ribonuclease, the embryo extract of the present invention usually contains an amount of tRNA that is sufficient for the protein synthesis reaction, which eliminates the necessity of adding separately prepared tRNA, as was conventional. Embryo extract of this sort functions as cell-free protein synthesis solution at least comprising a plant embryo extraction product, and in the presence of components necessary or suitable for protein synthesis, such as ATP, GTP, creatine phosphate, creatine kinase, L-amino acids, potassium ions, magnesium ions, and the like, it has sufficient protein synthesis activity, without tRNA being added. Herein, a cell-free protein synthesis solution that "has sufficient protein synthesis activity without tRNA being added" means, as described in the examples below, a cell-free protein synthesis solution which, even when tRNA is not added, has a protein synthesis activity that is at least equivalent to the protein synthesis activity of conventional protein synthesis solutions, wherein a plant embryo which has been frozen in liquid nitrogen or the like is milled or crushed in a mortar or the like, and subsequently extracted so as to prepare a embryo extract to which a sufficient amount (an amount whereby the protein synthesis reaches a plateau) of tRNA is added. More preferably the solution is a solution that, when no tRNA is added, has a protein synthesis activity equal to, or greater than, the protein synthesis activity when tRNA is added.

Furthermore, an aqueous solution containing the embryo extract of the present invention, having sufficient protein synthesis activity without tRNA being added as described above, and components necessary or suitable for protein synthesis, such as ATP, GTP, creatine phosphate, creatine kinase, L-amino acids, potassium ions, magnesium ions, and the like, can easily be used as a ready-made, cell-free protein synthesis solution. There are no particular restrictions on the amounts of the aforementioned components contained so long as the concentrations allow the cell-free protein synthesis reaction to be performed. Such a ready-made, cell-free protein synthesis solution allows for large amounts of protein to be synthesized simply and efficiently, simply by adding the target translation template (mRNA), without preparing a reaction solution for the protein synthesis, as was conventional. The protein synthesis solution can be prepared using the aforementioned extracting solvent as a solvent and adding the aforementioned components as necessary.

A kit for performing the cell-free protein synthesis of the present invention comprises at least the embryo extract or the cell-free protein synthesis solution described above, and may comprise optional elements such as other reagents and reaction vessels necessary to the cell-free protein synthesis, including diluents (buffer solution), dialysates, energy sources, expression vectors, expression vectors for positive controls, amino acids as substrate, dialysis tubes, and the like. Furthermore, the reagent kit may contain reagents used in transcription systems such as RNA polymerase.

### Examples

In the following, the present invention is described in further detail by way of examples, but the following examples are only intended to aid in concrete appreciation of the present invention, and the scope of the present invention is in no way limited to the examples described below.

Note that in the following examples: I stands for liter; ml stands for milliliter; M stands for mol/liter; mM stands for millimol/liter, and $\mu$g stands for microgram.

Example 1: pulverization and extraction using a Waring blender (1)

Hokkaido Chihoku wheat (undisinfected) was added to a mill (Pulverisette 14 Rotor Speed Mill, Fritsch) at a rate of 100 g per minute, and the grains were moderately ground at 7000 rpm. This grinding process was repeated four times. After recovering a fraction containing germinatable embryos with a sieve (mesh size 0.71 to 1.00 mm), the surfacing fraction containing the germinatable embryos was recovered by heavy medium separation using a mixture of carbon tetrachloride and cyclohexane (volume ratio = carbon tetrachloride : cyclohexane = 2.4 : 1), the organic solvent was eliminated by desiccation at room temperature, and then impurities such as seed coat were eliminated by air-blowing at room temperature to obtain a crude embryo fraction.

Next, a belt type color sorter BLM-300K (Manufacturer: Anzai Manufacturing Co., Ltd., Marketed by: Anzai Co., Ltd.) was used to select the embryo from the crude embryo fraction by way of color difference. This color sorter is a device comprising: means for irradiating the crude embryo fraction with light; means for detecting reflected light and/or transmitted light from the crude embryo fraction; means for comparing the detected value with a reference value; and means for selecting and eliminating that which is outside the standard value or that which is within the standard value.

The crude embryo fraction was supplied onto the color sorter belt so as to produce 1,000 to 5,000 particles/$m^2$, the crude embryo fraction on the belt was irradiated with fluorescent light and the reflected light was detected. The belt transport speed was 50 m/minute. A monochrome CCD line sensor (2048 pixels) was used as the photosensor.

First, in order to eliminate components darker than the embryo (seed coat and the like), a beige colored belt was used and the standard value was set between the brightness of the embryo and seed coat and objects outside of the standard value were removed by suctioning. Next, in order to select the endosperm, a dark green belt was used and the standard value was set between the brightness of the embryo and endosperm, and objects outside of the standard value were removed by suctioning. Suctioning was performed by way of 30 suction nozzles (the suction nozzles were aligned with one suction nozzle for each centimeter of length) positioned approximately 1 cm above the transport belt.

By repeating this process, the embryo was selected to a embryo purity (weight ratio of embryo per gram in any sample) of no less than 98%.

Fifty grams of the wheat embryo obtained as described above were suspended in distilled water at 4°C and this was washed with an ultrasonic cleaner until the wash solution was no longer clouded. Next, this was suspended in a 0.5% by volume solution of Nonidet P40, and washed with an ultrasonic cleaner until the wash solution was no longer clouded, so as to produce wheat embryo from which the endosperm had been removed.

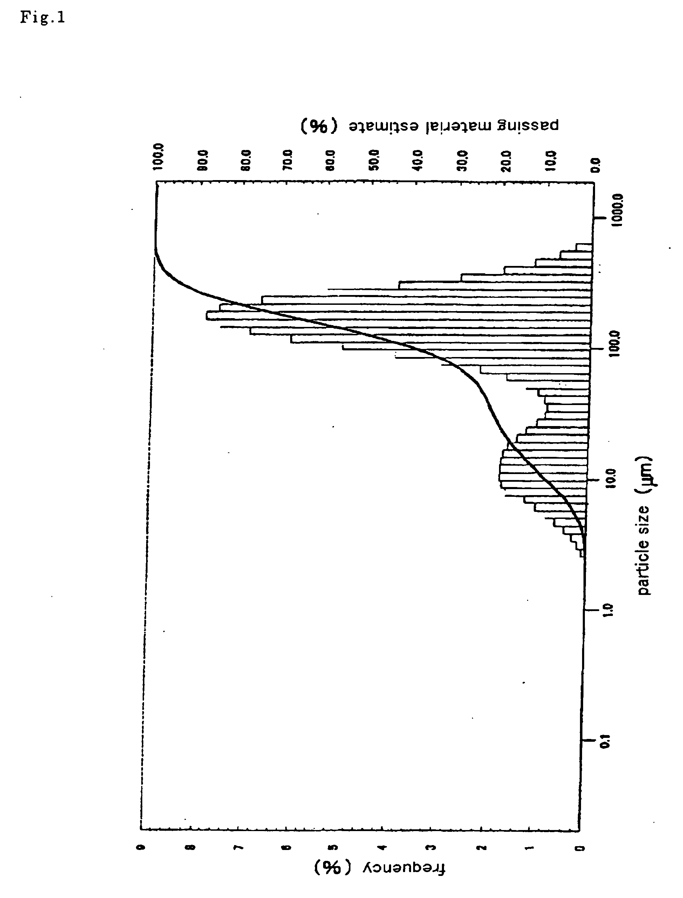

Next, the following operations were performed at 4°C to produce a embryo extract (solution containing wheat embryo extraction products). First, the washed wheat embryo was placed in a Waring blender together with 100 mM of extracting solvent (80 mM of HEPES-KOH [pH7.6], 200 mM of potassium acetate, 10 mM of magnesium acetate, 4 mM of calcium chloride, 0.6 mM each of the 20 kinds of L-amino acids and 8 mM of dithiothreitol) and ground for 30 seconds at 5,000 to 20,000 rpm. Twice thereafter, the embryo which had adhered to the inner walls of the blender was scraped off and the grinding was repeated for 30 seconds at 5,000 to 20,000 rpm. The particle size distribution of the ground embryo produced was measured with a laser-scattering granulometer (LA-920, Horiba, Ltd.). The results are shown in Figure 1.

The resulting mixture of extract solution and ground embryo was transferred to a centrifuge tube, centrifuged for 30 minutes at 30,000 g, and the supernatant was recovered. The operation of centrifuging for 30 minutes at 30,000 g and collecting the supernatant was repeated five times so as to obtain an unclouded supernatant. Gel filtration was performed using a Sephadex G-25 column that had been pre-equilibrated with a solution consisting of 40 mM of HEPES-KOH (pH7.6), 100 mM of potassium acetate, 5 mM of magnesium acetate, 0.3 mM each of the 20 types of L-amino acids and 4 mM of dithiothreitol. The resulting solution was centrifuged for 12 minutes at 30,000 g and the supernatant was recovered to produce a solution containing wheat embryo extraction product. The sample concentration was adjusted with extracting solvent so that the optical density at 260 nm (O.D.) ($A_{260}$) was 80 to 150 ($A_{260}/A_{280}$ = 1.5).

Example 2: preparation of a wheat embryo cell-free protein synthesis system using dialysis

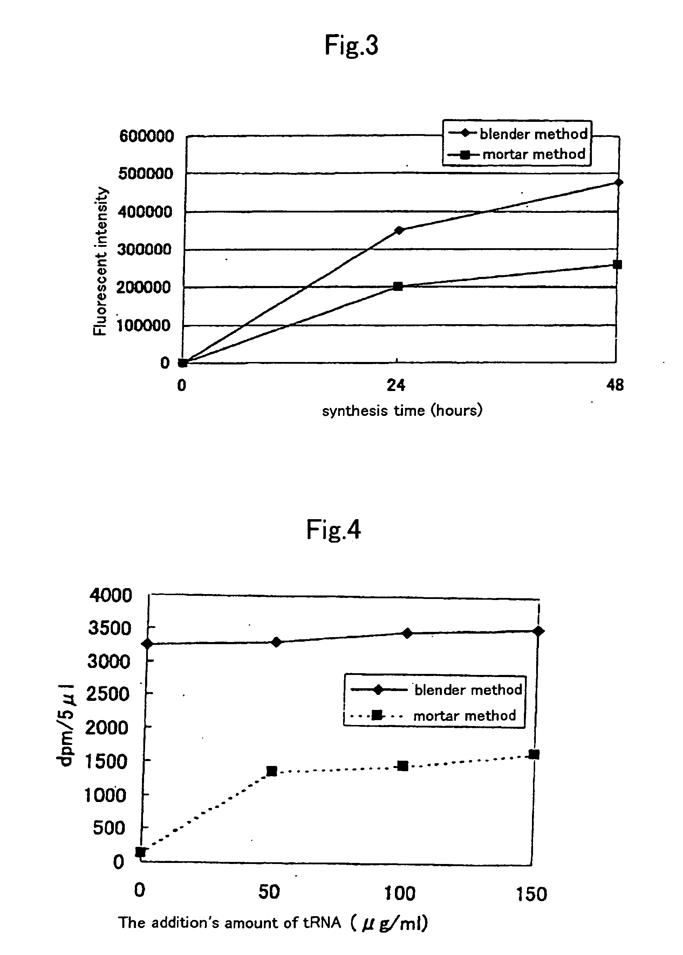

The concentration of the solution containing wheat embryo extraction product produced in Example 1 was adjusted with extracting solvent so that the optical density at 260 nm (O.D.) ($A_{260}$) was 90 and green fluorescent protein (GFP) was synthesized according to the method described in Endo, Y. et al., PNAS, January 18, 2000, Vol. 97, No. 2, 559-564. The GFP activity was quantified by measuring the 510 nm fluorescent intensity at an excitation wavelength of 490 nm using a TD-360 Mini-Fluorometer by Turner Designs. As shown in Figure 3 (indicated as blender method in Figure 3) the fluorescent intensity was measured to be approximately 350,000 after 24 hours and 480,000 after 48 hours, confirming that GFP was being synthesized.

Example 3: analysis of solution containing wheat embryo extraction product (1)

Hokkaido Chihoku wheat (undisinfected) was used to prepare a solution containing wheat embryo extraction product by the same method as in Example 1. The concentration of the samples were adjusted with extracting solvent so that the optical density at 260 nm (O.D.) ($A_{260}$) was 90, and the content of DNA and RNA in each of the samples was measured. The results are shown below.

|           | RNA ($\mu$g/ml) | DNA ($\mu$g/ml) |
|-----------|-----------|-----------|
| Sample a1 | 1411      | 141       |
| Sample a2 | 1554      | 142       |

The methods for measuring the DNA and RNA content are as follows.

Method for measuring DNA content

DNA content was measured using a microplate fluorophotometer (SPECTRAmax GEMINI XS, Molecular Devices) using PicoGreen dsDNA quantitation reagent (Molecular Probes), with Calf Thymus DNA Standard (Pharmacia Biotech) as a standard sample.
First, 10 $\mu$l of proteinase K (10 mg/ml) were added to 200 $\mu$l of sample and this was reacted overnight at 55°C. After extracting the reaction solution with an equal volume of phenol, this was deproteinized by further extraction with an equal volume of phenol/chloroform (1:1) and ethanol precipitation was performed by adding a 1/10 volume of 3 M sodium acetate and 2 volumes of ethanol. After washing with 70% ethanol, this was dissolved in 10 mM of tris hydrochloride buffer (pH 8.0) (TE) containing 1 mM sodium ethylenediaminetetraacetate. RNase A (10 mg/ml) in the amount of 5 $\mu$l was added to the solution and reacted overnight at 37°C. This was extracted with phenol and phenol/chloroform as described above to remove the RNA. The sample was ethanol precipitated, washed and dissolved in 200 $\mu$l of TE. Picogreen dsDNA quantitation reagent, diluted 200 fold with TE, and the sample, which had been diluted 100 fold with TE, were mixed at a 1:1 ratio, the fluorescence was measured at 485/530 nm excitation/emission (fluorescent) wavelengths, and the DNA content was calculated using a standard curve.

Method for measuring RNA content

RNA content was measured using a microplate fluorophotometer (SPECTRAmax GEMINI XS, Molecular Devices) using BioGreen RNA Quantitation reagent (Molecular Probes) as a fluorescence reagent, with Ribosomal RNA standard (16S and 23S rRNA from *E. coli*) as the standard RNA.
First, 3 $\mu$l of DNase I (RNase free, 1 U/$\mu$l) were added to 100 $\mu$l of the extract solution, and this was reacted for 30 minutes at 37°C. Purified water in the amount of 300 $\mu$l was added to the reaction solution and after extracting this twice with 400 $\mu$l of water-saturated phenol, this was further extracted with 400 $\mu$l of chloroform to remove the DNA. To this were added a 1/10 volume of 3M sodium acetate and 2 volumes of ethanol, for ethanol precipitation, and this was dissolved in 100 $\mu$l of TE. To 100 $\mu$l of the sample, which had been diluted 1,500 fold with TE, was added 100 $\mu$l

of 200 fold diluted BioGreen RNA Quantitation reagent and fluorescence was measured at 480/520 nm excitation/ emission (fluorescent) wavelengths, and the RNA content was calculated using a standard curve.

Example 4: Analysis of the solution containing wheat embryo extract product (2)

Hokkaido Chihoku wheat (undisinfected) was used to prepare a solution containing wheat embryo extraction product by the same method as in Example 1. The concentration of the samples were adjusted with extracting solvent so that the optical density at 260 nm (O.D.) ($A_{260}$) was 90, and the content of the lipids (acid hydrolysis) and total fatty acid (gas chromatography) in each of the samples was measured. The results are shown below.

|  | lipids (g/100g) | total fatty acids (g/100g) |
|---|---|---|
| Sample a3 | below detection limit | 0.03 |
| Sample a4 | below detection limit | 0.03 |

The method for measuring the lipid content is as follows.

Method for measuring lipid content (acid hydrolysis)

A sample (S) in the amount of 5.0 g and 10 ml of concentrated hydrochloric acid were placed in a container, and the acid hydrolysis was performed by heating to 80°C in a hot water bath for 40 minutes. The digestion product produced was transferred to a Majonia tube with ethyl ether and extracted by shaking with a mixed solution of diethyl ether/petroleum ether (volume ratio of 1:1). This was washed with ion-exchanged water until the ether layer ceased to show acidity. The ether layer was recovered and transferred to a weighing vessel ($W_1$ g), and after removing the ether, this was further desiccated for one hour at 105°C and weighed ($W_2$ g). The lipid content (g/100 g) was calculated according to the following formula.

$$\text{Lipid content (g/100g)} = (W_2 - W_1) \div S \times 100$$

Method for measuring total fatty acid content (gas chromatography)

Acid hydrolysis was performed by adding 2 mg of heptadecanoic acid (internal standard), 4 ml of ethanol, 6.7 ml of ion-exchanged water and 8.3 ml of 12 mol/l hydrochloric acid to 5.0 g of sample. Next, 16 ml of ethanol was added, extraction was performed by adding 100 ml of a mixture of diethyl ether/petroleum ether (volume ratio 1:1), and this was twice extracted with 60 ml of this mixture. After washing with water to remove the extracting solvent, saponification and methylesterification were performed according to the ACOS Official Method Ce lb-89 (1997), whereafter measurements were performed by gas chromatography under the following operating conditions.

*Gas chromatography conditions*

Measurement device: Shimadzu GC-17A

Detector: FID

Column: J & W DB-23, $\phi$ 0.25 mm x 30 mm, df. 0.25 $\mu$m

Column temperature: 70°C (1 minute retention) → 170°C (Temperature rise: 10°C/min) → 210°C (Temperature rise: 1.2°C/min)

Inlet temperature: 250°C

Detector temperature: 250°C

Gas flow: helium 1.5 ml/minute

Gas pressure: hydrogen 60 kPa, air 50 kPa

Injector: splitless

The contents of the various fatty acids (palmitic acid, oleic acid and linoleic acid) were calculated based on the readings obtained according to the following formula, so as to determine the total fatty acid content.

$$\text{Content in each fatty acid (g/100g)} = (E \times F \times H/D \times G) \times 0.1$$

(In the formula, D represents the peak area for the heptadecanoic acid, E represents the peak area of each of the fatty acids, F represents the amount of heptadecanoic acid added (mg), G represents the amount of sample used and H represents the sensitivity correction coefficient, which is the previously measured sensitivity of each fatty acid to heptadecanoic acid.)

Example 5: analysis of solution containing wheat embryo extraction product (3)

Okayama Shirasagi wheat (undisinfected) was used to prepare a solution containing wheat embryo extraction product by the same method as in Example 1, the concentration of the samples were adjusted with extraction buffer solution so that the optical density at 260 nm (O.D.) ($A_{260}$) was 90, and the content of DNA and RNA was measured by the same method as in Example 3.

The results are shown below.

|  | RNA ($\mu$g/ml) | DNA ($\mu$g/ml) |
|---|---|---|
| Sample a5 | 1401 | 123 |

Comparative Example 1: frozen pulverization extraction using a mortar (1)

Using the same method as in Example 1, Hokkaido Chihoku wheat (undisinfected) was ground and separated to produce a crude embryo fraction and the embryo was selected to a embryo purity of no less than 98% using the color sorter. Furthermore, the embryo was washed and the endosperm removed by the same method as in Example 1 to produce wheat embryo.

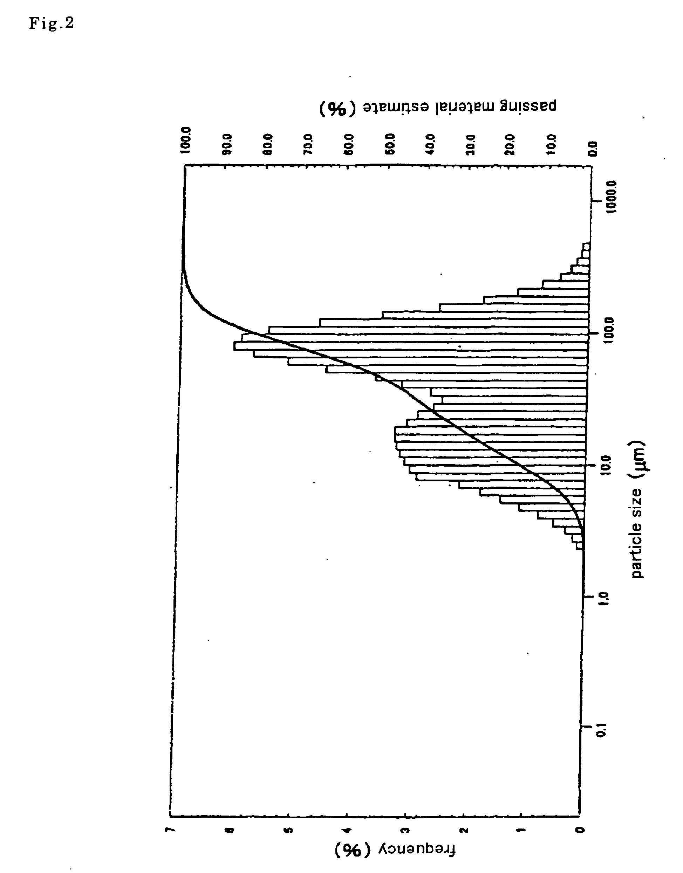

Next, the following operations were performed at 4°C to produce a solution containing wheat embryo extraction product. First, the washed wheat embryo was frozen in liquid nitrogen and pulverized in a mortar. The particle size distribution of the resulting ground embryo is shown in Figure 2.

The extracting solvent (80 mM of HEPES-KOH (pH 7.6), 200 mM of potassium acetate, 10 mM of magnesium acetate, 4 mM of calcium chloride, 0.6 mM of each of the 20 types of L-amino acids and 8 mM of dithiothreitol) in the amount of 10 ml were added to the mortar and mixed. The resulting mixture of extract solution and ground embryo was transferred to a centrifuge tube, centrifuged for 30 minutes at 30,000 g, and the supernatant was recovered. This was further centrifuged under the same conditions as in Example 1 to produce an unclouded supernatant, and after gel filtration, this was centrifuged to produce a supernatant, which was a solution containing wheat embryo extraction product. The sample concentration was adjusted with extracting solvent so that the optical density at 260 nm (O.D.) ($A_{260}$) was 170 to 250 ($A_{260}/A_{280}$ = 1.5).

Comparative Example 2: preparation of dialysis-based wheat embryo cell-free proteins synthesis system

The concentration of the solution containing wheat embryo extraction product obtained in Example 1 was adjusted with extracting solvent so that the optical density at 260 nm (O.D.) ($A_{260}$) was 90, and Green fluorescent protein (GFP) was synthesized according to the method described in Endo, Y. et al., PNAS, January 18, 2000, Vol. 97, No. 2, 559-564. The GFP activity was quantified by measuring the 510 nm fluorescent intensity at an excitation wavelength of 490 nm using a TD-360 Mini-Fluorometer by Turner Designs. As shown in Figure 3 (indicated as mortar method in Figure 3), the fluorescent intensity was observed to be approximately 200,000 after 24 hours, and approximately 260,000 after 48 hours, confirming that GFP was being synthesized, but the values were lower than those in Example 2.

Comparative Example 3: analysis of solution containing wheat embryo extraction product (1')

Hokkaido Chihoku wheat (undisinfected) was used to prepare a solution containing wheat embryo extraction product by the same method as in Comparative Example 1. The concentration of the samples were adjusted with extraction solvent so that the optical density at 260 nm (O.D.) ($A_{260}$) was 90, and the content of DNA and RNA in each of the samples was measured by the same method as in Example 3. The results are shown below.

| | RNA ($\mu$g/ml) | DNA ($\mu$g/ml) |
|---|---|---|
| Sample b1 | 1567 | 283 |
| Sample b2 | 1574 | 283 |
| Sample b3 | 1578 | 232 |

As is made clear from the results above, the RNA content was approximately equal to the value for the solution containing embryo extraction product obtained in Example 3 (the ratio of sample a1 to sample b1 being approximately 1.1 times) and the DNA content was approximately double.

Comparative Example 4: analysis of solution containing wheat embryo extraction product (2')

Hokkaido Chihoku wheat (undisinfected) was used to prepare a solution containing wheat embryo extraction product by the same method as in Comparative Example 1. The concentration of the samples were adjusted with extracting solvent so that the optical density at 260 nm (O.D.) ($A_{260}$) was 90, the lipid (acid hydrolysis) and fatty acid (gas chromatography) content in each of the samples was measured by the same method as in Example 4. The results are shown below.

| | lipids (g/100g) | total fatty acids (g/100g) |
|---|---|---|
| Sample b4 | 0.1 | 0.06 |
| Sample b5 | 0.05 | 0.05 |

As is made clear from the results above, the lipid and total fatty acid contents were both higher than the content values for the solution containing embryo extraction product obtained in Example 4. The total fatty acid content was double, when comparing sample a3 and sample b4.

Example 6: pulverization extraction using a Waring blender (2)

Hokkaido Chihoku wheat (undisinfected) was used and a solution containing wheat embryo extraction product was produced by the same method as in Example 1, other than the fact that an extracting solvent that did not contain calcium chloride (80 mM of HEPES-KOH[pH7.8], 200 mM of potassium acetate, 10 mM of magnesium acetate, 0.6 mM each of the 20 types of L-amino acids and 8 mM of dithiothreitol) was used as the embryo extracting solvent. The sample concentration was adjusted with extracting solvent so that the optical density at 260 nm (O.D.) ($A_{260}$) was 80 to 150 ($A_{260}/A_{280}$ = 1.5).

Example 7: wheat embryo cell-free protein synthesis reaction using the batch method (1)

The concentration of the solution containing wheat embryo extraction product obtained in Example 6 was adjusted with extracting solvent so that the optical density at 260 nm (O.D.) ($A_{260}$) was 110 and the reaction solution was prepared, and the reaction of itself was performed, according to a known method (Madin, K. et al., Proc. Natl. Acad. Sci. USA, 2000, 97, 559-564) which was partially modified. That is to say, a reaction solution having the composition shown below in (*1) was prepared.

DHFR mRNA (with no cap structure) prepared by transcribing a dihydrofolate dehydrogenase (DHFR) gene incorporated in an expression vector (pEU), which is specifically intended for use in wheat embryo cell-free protein synthesis systems, was used as a model translation template (WO 01/27260), 60 $\mu$g/ml were added and the synthesis reaction was performed at 26°C. The synthesis activity was measured based on the [$^{14}$C]-leucine incorporated into the protein in 5 $\mu$l of reaction solution after two hours of reaction at 26°C.

Here, in order to determine whether or not addition of tRNA was necessary, synthesis was performed according to four different cases wherein 0 μg/ml, 50 μg/ml, 100 μg/m, and 150 μg/ml of tRNA were added, as a result of which it was understood that sufficient synthesis activity was achieved even without the addition of tRNA (indicated as blender method in Figure 4). Furthermore, these results also made it clear that it was possible to produce a solution containing embryo extraction product having sufficient protein synthesis activity, even when there was no calcium in the extraction solvent.

<u>(*1) Composition of the solutions for wheat embryo cell-free protein synthesis</u>

The reaction solutions comprise 24% by volume of wheat embryo extract having an optical density of 200 at 260 nm (O.D.) ($A_{260}$) (and thus, 43.5% with an optical density of 110 at 260 nm [O.D.] [$A_{260}$]) and has the following component composition. 30 mM of HEPES-KOH (pH 7.6), 95 mM of potassium acetate, 2.65 mM of magnesium acetate, 2.85 mM of dithiothreitol, 1.2 mM of adenosine triphosphate (ATP), 0.25 mM of guanosine triphosphate (GTP), 16 mM of creatine phosphate, 0.5 mg/ml of creatine kinase, 0.380 mM of spermidine, 20 kinds of L-amino acids (0.3 mM each), 1,000 units/ml of ribonuclease inhibitor (RNase inhibitor).

<u>Comparative Example 5: Frozen pulverization extraction with a mortar (2)</u>

Hokkaido Chihoku wheat (undisinfected) was used and a solution containing wheat embryo extraction product was produced by the same method as in Example 1, other than the fact that the extracting solvent that did not contain calcium chloride (80 mM of HEPES-KOH (pH7.8), 200 mM of potassium acetate, 10 mM of magnesium acetate, 0.6 mM of each of the 20 types of L-amino acids, and 8 mM of dithiothreitol) was used as the embryo extracting solvent. The sample concentrations were adjusted with extracting solvent so that the optical density at 260 nm (O.D.) ($A_{260}$) was 170 to 250 ($A_{260}/A_{280}$ = 1.5).

<u>Comparative Example 6: wheat embryo cell-free protein synthesis reaction using the batch method</u>

The concentration of the solution containing wheat embryo extraction product obtained in Comparative Example 5 was adjusted so that the optical density at 260 nm (O.D.) ($A_{260}$) was 210, the reaction solution was prepared in the same manner as in Example 7, protein was synthesized, and the [$^{14}$C]-leucine that was incorporated into the protein was measured. Note that reaction solutions were used comprising 24% by volume of wheat embryo extract that had an optical density of 200 at 260 nm (O.D.) ($A_{260}$) (and thus, 22.8% with an optical density of 210 at 260 nm [O.D.] [$A_{260}$]).
As is made clear by Figure 4, as in Example 7, synthesis was performed according to four different cases wherein 0 μg/ml, 50 μg/ml, 100 μg/m, and 150 μg/ml of tRNA were added, as a result of which it was understood that sufficient synthesis activity was not achieved when tRNA was not added (indicated as mortar method in Figure 4).

<u>Example 8: measurement of ribonuclease activity</u>

(1) Embryo Extract

The embryo extract of the present invention (Example 1) and extract produced according to conventional methods (Comparative Example 1) were used as the wheat embryo extracts for measurement of ribonuclease activity. In order to measure the activity, a conventional embryo extract was used, which contained 30 mM of Hepes-KOH (pH 7.8), 1.2 mM of ATP, 0.25 mM of GTP, 16 mM of creatine phosphate, 2 mM of dithiothreitol, 0.3 mM spermidine, 0.3 mM of the 20 kinds of amino acids, 2.5 mM of magnesium acetate, 100 mM of potassium acetate, 0.005% of sodium azide (hereinafter referred to as D.B.) and which was applied to a MicroSpin G-25 Column. The extract of the present invention contained D.B. The concentrations of the two embryo extracts (the embryo extract of the present invention and the embryo extract produced by conventional extraction) were made equal based on $OD_{260}$ measurement values.

(2) Preparation of the Reaction Solution

pEU-DHFR (see WO 01/27260) was subject to PCR amplification using a sense primer and an antisense primer and this was used as a template in transcription that produced mRNA, which was used as the substrate for the ribonuclease. The transcription was performed with a 400 μl reaction system, which was prepared so as to contain 80 mM of Hepes-KOH, 16 mM of magnesium acetate, 2 mM of spermidine, 10 mM of DTT, 3 mM of NTP, 1U/μl of SP6RNA polymerase, 1 U/μl of RNase inhibitor (RNasin) and 10% PCR product, and which was incubated for three hours at 37°C. In cases where the mRNA was labeled with radioisotope $^{32}$P, 8 μl of [α-$^{32}$P] UTP was added to a 400 μl reaction system having a UTP concentration of 1.2 mM. After incubation, the solution was centrifuged for 2 minutes at 10,000 g, 53 μl of 7.5 M ammonium acetate and 1 ml of ethanol were added to the supernatant, and this was centrifuged for 15 minutes at 20,000 g. The precipitate was washed with 1 ml of 70% ethanol, whereafter RNA dissolved in 80 $\mu$l of D.B. was used. The various compositions are as shown below.

mRNA (no radioisotope)

| 5 x TB | 80 $\mu$l |
|---|---|
| 25 mM of NTP | 48 $\mu$l |
| SP6 RNA polymerase | 5 $\mu$l |
| RNase inhibitor | 5 $\mu$l |
| DNA | 40 $\mu$l |
| Milli-Q water | 222 $\mu$l |
| | 400 $\mu$l |

mRNA (with radioisotope)

| 5 x TB | 80 $\mu$l |
|---|---|
| 25 mM of ATP CTP GTP 10 mM of UTP | 48 $\mu$l |
| [$\alpha$-$^{32}$P] UTP | 8 $\mu$l |
| SP6 RNA polymerase | 5 $\mu$l |
| RNase inhibitor | 5 $\mu$l |
| DNA | 40 $\mu$l |
| Milli-Q water | 214 $\mu$l |
| | 400 $\mu$l |

(3) RNA Degradation Control (assay using RNase A as a standard preparation)

As an RNA degradation control, variously, 30 pg, 300 pg, 3 ng and 30 ng of RNase A were added to 2 $\mu$l of mRNA (no radioisotope) and 1 $\mu$l of mRNA (with radioisotope) and the reaction systems were adjusted to 30 $\mu$l with D.B. and incubated for 30 minutes at 26°C. The various compositions are as shown below.

| mRNA (no radioisotope) | 2 $\mu$l | 2 $\mu$l | 2 $\mu$l | 2 $\mu$l | 2 $\mu$l |
|---|---|---|---|---|---|
| mRNA (with radioisotope) | 1 $\mu$l | 1 $\mu$l | 1 $\mu$l | 1 $\mu$l | 1 $\mu$l |
| RNase A D. B. | - | 30 pg | 300 pg | 3 ng | 30 ng |
| Total | 30 $\mu$l | 30 $\mu$l | 30 $\mu$l | 30 $\mu$l | 30 $\mu$l |

For ribonuclease activity in the embryo extract of the present invention and the embryo extract resulting from conventional methods, 2 $\mu$l of mRNA (no radioisotope) and 1 $\mu$l of mRNA (with radioisotope) were added to the reaction systems, together with 8 $\mu$l or 12 $\mu$l of the extracts, so that the final densities were 40 OD$_{260}$/ml, and these were adjusted to 30 $\mu$l with D.B. Four tubes of each were prepared and incubated at 26°C.

| | Embryo extract of the present invention | Embryo extract resulting from conventional methods |
|---|---|---|
| mRNA (no radioisotope) | 2 $\mu$l | 2 $\mu$ |
| mRNA (with radioisotope) | 1 $\mu$ | 1 $\mu$ |
| embryo extract | 8 $\mu$ | 12 $\mu$ |
| D. B. | 19 $\mu$ | 15 $\mu$ |
| Total | 30 $\mu$ | 30 $\mu$ |

After incubation for 10 minutes and 30 minutes, these were frozen with liquid nitrogen to stop the reaction. Milli-Q water in the amount of 255 $\mu$l, 15 $\mu$l of 10% SDS, and 300 $\mu$l of phenol/Milli-Q water were added, and this was vortexed for 10 minutes, and centrifuged for 10 minutes at 20,000 g. An amount of 300 $\mu$l of the aqueous phase was transferred to a new tube, 300 $\mu$l of phenol/Milli-Q was added, and this was vortexed for 10 minutes, and centrifuged for 10 minutes at 20,000 g. An amount of 300 $\mu$l of the aqueous phase was transferred to a new tube, 300 $\mu$l of chloroform was added, and this was vortexed for 10 minutes, and centrifuged for 1 minute at 20,000 g. An amount of 300 μl of the aqueous phase was transferred to a new tube, 30 μl of 5 M sodium chloride and 750 μl of ethanol were added, and this was centrifuged for 15 minutes at 20,000 g. The precipitate was washed in 1 ml of 70% ethanol and dissolved in 54 μl of Milli-Q water. An amount of 6 μl of 10 x Sample buffer (0.25% Bromophenol blue, 50 mM of Tris-HCl pH7.6, 6% glycerol) was added and a 5 μl sample was electrophoresed on 1.2% agarose. The presence or absence of RNA degradation was verified by way of an autoradiogram (FUJIFILM: BAS-2500). The results are shown in Figure 5. Note that M indicates a marker. (A) is a graph showing the RNA degradation when an mRNA (substrate) is treated with a solution containing RNase A, prepared at four levels of concentration. (B) is a graph showing the RNA degradation when an mRNA (substrate) is treated with the embryo extract of the present invention, and separately with embryo extract produced by extraction according to conventional methods.

In RNase A treatment, the mRNA was almost entirely degraded by 100 pg/ml of RNase A. The band intensities for the embryo extract of the present invention and the conventional embryo extract were calculated based on the PLS using ImageGauge (FUJIFILM: BAS2500), and taking the RNase A untreated sample as 100%. The results are shown in Figure 6. It was understood that the ribonuclease content of the extract of the present invention was no greater than 10 pg/μl as converted to RNase A.

It is not absolutely clear why a embryo extract having the excellent characteristics described above can be produced by means of the present invention but the reasons may be as follows.

A system for protein synthesis in a plant embryo (ribosomes, tRNA, aminoacyl-tRNA synthetases, translation factors, and the like) is prepared for active protein synthesis, which begins in accordance with the supply of mRNA, which migrates to the cytoplasm after being transcribed in the cell nucleus during germination, but all of these factors are localized in cytoplasm alone, where the translation reaction takes place. In other words, the factors that contribute to protein synthesis are not found in components other than the cytoplasm (cellular membranes, cell walls, organelles such as the cell nucleus, or the like, of the embryo). Cellular membranes, cell walls, organelles such as the cell nucleus, and the like contain unnecessary DNA, lipids and the like, which do not contribute to protein synthesis. Thus, solutions containing embryo extraction product produced by conventional methods, wherein frozen embryo was pulverized by milling or crushing with a mortar, stamp mill, bowl mill or the like, and thereafter extracted with an extracting solvent, contain large quantities of these unnecessary components which do not contribute to the protein synthesis reaction.

Components which do not contribute to the protein synthesis reaction include those which inhibit the protein synthesis reaction and those having a disruptive effect on Higher-Order Structure Formation and functional analysis of synthesized products. For example, DNA is an acidic polymer that binds to basic proteins such as ribosome proteins and inhibits the translation process. It is also possible that during synthesis of DNA binding proteins, some embryo-derived DNA fragments have base sequences that bind strongly thereto. Furthermore, lipid components often interfere with the protein purification process and it is generally necessary to eliminate them in a preprocessing operation. In addition, it is possible that some low molecular weight substances have an inhibitory effect on protein synthesis.

Furthermore, contamination of ribonuclease results in degradation of template mRNA, while contamination of phosphatase results in degradation of energy sources such as ATP.

The present invention provides a embryo extract (which is useful in cell-free protein synthesis systems) wherein the embryo is minced in the presence of the extracting solvent, or wherein the embryo is minced by impact or chopping whereafter extraction is performed by adding extracting solvent. By employing the novel technique of mincing embryo by impact or chopping, which was not conventionally used, cellular membranes, cell walls, organelles such as the cell nucleus, and the like, are not broken down more than is necessary, whereby it is possible to avoid the conventional method's problem of extracting components that were not necessary to, or which had a negative impact on, the protein synthesis reaction. Furthermore, even in cases where the embryo extract is prepared employing such conventional methods as mincing the embryo by milling or crushing, by performing this mincing in the presence of the extracting solvent, extraction can be performed more rapidly, and as a consequence, cellular membranes, cell walls, organelles such as the cell nucleus, and the like, are not broken down more than is necessary, whereby it is again possible to avoid the conventional method's problem of extracting components that were not necessary to, or which had a negative impact on, the protein synthesis reaction.

## Possibilities for industrial use

By virtue of the present invention, it is possible to efficiently extract factors necessary for protein synthesis from the cytoplasm and produce a embryo extract low in impurities such as DNA and lipids, which are contained in cellular membranes, cell walls, organelles such as the cell nucleus, and the like, allowing for stable provision of a embryo extract for high-efficiency cell-free protein synthesis and a cell-free protein synthesis solution. The use of the embryo extract for protein synthesis, or the solution for protein synthesis, of the present invention makes it possible to stably achieve high-efficiency cell-free protein synthesis. Furthermore, as it is possible to prepare large quantities of high purity embryo extract in a short period of time, this is extremely useful in large-scale production of cell-free protein systems, such as the large-scale production of novel enzymes and antibodies in the field of evolutionary molecular engineering.

**Claims**

1. A cell-free protein synthesis method comprising:

   - preparing plant embryo extract comprising the steps of:

     (a)

        (1) mincing a plant embryo by chopping, and
        (2) extracting the minced plant embryo with an extracting solvent,

     or
     (a)' mincing a plant embryo in the presence of extracting solvent by chopping and extracting the plant embryo which has been minced, wherein said steps of mincing and extracting are carried out simultaneously, and

   - using the plant embryo extract in cell-free protein synthesis.

2. The cell-free protein synthesis method according to claim 1, wherein the mincing of a plant embryo by chopping is by means of a high speed rotary blade.

3. The cell-free protein synthesis method according to any of claims 1 or 2, wherein the step of mincing the plant embryo is in the presence of extracting solvent.

4. The cell-free protein synthesis method according to any of claims 1 to 3, wherein the extracting solvent comprises at least one substance selected from the group of: a buffer solution, potassium ions, magnesium ions, and a thiol antioxidant.

5. The cell-free protein synthesis method according to any one of claims 1 to 4, wherein the plant embryo is the embryo of wheat, barley, rice or corn.

6. The cell-free protein synthesis method according to any one of claims 1 to 5, wherein the plant embryo is substantially uncontaminated by an endosperm component.

7. A cell-free protein synthesis solution **characterised by** comprising :

   - an embryo extract produced by a preparation method comprising the steps of:

     (a)

        (1) mincing a plant embryo by chopping, and
        (2) extracting the minced plant embryo with an extracting solvent,

     or
     (a)' mincing a plant embryo in the presence of extracting solvent by chopping and extracting the plant embryo which has been minced, wherein said steps of mincing and extracting are carried out simultaneously,

     wherein the plant embryo is substantially uncontaminated by an endosperm component,
   - and ATP, GTP, creatine phosphate creatine kinase, L-amino acids, potassium ions and magnesium ions.

8. The cell-free protein synthesis solution according to claim 7, wherein the mincing of the plant embryo by chopping is by means of a high speed rotary blade.

9. The cell-free protein synthesis solution according to any of claims 7 or 8, wherein the step of mincing the plant embryo is in the presence of extracting solvent.

**10.** The cell-free protein synthesis solution according to any of claim 7 to 9, wherein the extracting solvent comprises at least one substance selected from the group of: a buffer solution, potassium ions, magnesium ions, and a thiol antioxidant.

**11.** The cell-free protein synthesis solution according to any of claims 7 to 10, wherein the plant embryo is the embryo of wheat, barley, rice or corn.

**12.** The cell-free protein synthesis solution according to any of claims 7 to 11, said cell-free protein synthesis solution having sufficient protein synthesis activity without tRNA being added.

**13.** A kit for performing cell-free protein synthesis **characterised by** comprising the cell-free protein synthesis solution according to any of claims 7 to 12.

**Patentansprüche**

**1.** Zellfreies Proteinsyntheseverfahren umfassend:

- Herstellen eines Pflanzenembryoextrakts umfassend die Schritte:

(a)

(1) Zerkleinern eines Pflanzenembryos durch Zerhacken, und
(2) Extrahieren des zerkleinerten Pflanzenembryos mit einem extrahierenden Lösungsmittel,

oder
(a)' Zerkleinern eines Pflanzenembryos in Anwesenheit eines extrahierenden Lösungsmittels durch Zerhacken und Extrahieren des Pflanzenembryos, der zerkleinert wurde, wobei die Schritte des Zerkleinerns und Extrahierens gleichzeitig ausgeführt werden, und

- Verwenden des Pflanzenembryoextrakts in zellfreier Proteinsynthese.

**2.** Zellfreies Proteinsyntheseverfahren nach Anspruch 1, wobei das Zerkleinern eines Pflanzenembryos durch Zerhacken mittels einer mit hoher Geschwindigkeit rotierenden Klinge geschieht.

**3.** Zellfreies Proteinsyntheseverfahren nach einem der Ansprüche 1 oder 2, wobei der Schritt des Zerkleinerns des Pflanzenembryos in Anwesenheit eines extrahierenden Lösungsmittels stattfindet.

**4.** Zellfreies Proteinsyntheseverfahren nach einem der Ansprüche 1 bis 3, wobei das extrahierende Lösungsmittel mindestens eine Substanz aus der Gruppe: eine Pufferlösung, Kaliumionen, Magnesiumionen und ein Thiol-Antioxidans umfasst.

**5.** Zellfreies Proteinsyntheseverfahren nach einem der Ansprüche 1 bis 4, wobei der Pflanzenembryo der Embryo von Weizen, Gerste, Reis oder Mais ist.

**6.** Zellfreies Proteinsyntheseverfahren nach einem der Ansprüche 1 bis 5, wobei der Pflanzenembryo im wesentlichen nicht durch eine Endospermkomponente kontaminiert ist.

**7.** Zellfreie Proteinsyntheselösung **dadurch gekennzeichnet, dass** sie enthält:

- einen Embryoextrakt, der mittels eines Herstellungsverfahrens umfassend die Schritte:

(a)

(1) Zerkleinern eines Pflanzenembryos durch Zerhacken, und
(2) Extrahieren des zerkleinerten Pflanzenembryos mit einem extrahierenden Lösungsmittel,

oder

**18**

(a)' Zerkleinern eines Pflanzenembryos in Anwesenheit eines extrahierenden Lösungsmittels durch Zerhacken und Extrahieren des Pflanzenembryos, der zerkleinert wurde, wobei die Schritte des Zerkleinerns und Extrahierens gleichzeitig ausgeführt werden, wobei der Pflanzenembryo im wesentlichen nicht mit einer Endospermkomponente kontaminiert ist,

hergestellt wurde
- und ATP, GTP, Kreatinphosphat, Kreatinkinase, L-Aminosäuren, Kaliumionen und Magnesiumionen.

**8.** Zellfreie Proteinsyntheselösung nach Anspruch 7, wobei das Zerkleinern des Pflanzenembryos durch Zerhacken mittels einer mit hoher Geschwindigkeit rotierenden Klinge geschieht.

**9.** Zellfreie Proteinsyntheselösung nach einem der Ansprüche 7 oder 8, wobei der Schritt des Zerkleinerns des Pflanzenembryos in Anwesenheit eines extrahierenden Lösungsmittels stattfindet.

**10.** Zellfreie Proteinsyntheselösung nach einem der Ansprüche 7 bis 9, wobei das extrahierende Lösungsmittel mindestens eine Substanz aus der Gruppe: eine Pufferlösung, Kaliumionen, Magnesiumionen und ein Thiol-Antioxidans enthält.

**11.** Zellfreie Proteinsyntheselösung nach einem der Ansprüche 7 bis 10, wobei der Pflanzenembryo der Embryo von Weizen, Gerste, Reis oder Mais ist.

**12.** Zellfreie Proteinsyntheselösung nach einem der Ansprüche 7 bis 11, wobei die zellfreie Proteinsyntheselösung ausreichende Proteinsyntheseaktivität aufweist, ohne dass tRNA hinzugefügt wird.

**13.** Kit zur Durchführung von zellfreier Proteinsynthese **dadurch gekennzeichnet, dass** er die zellfreie Proteinsyntheselösung nach einem der Ansprüche 7 bis 12 umfasst.

**Revendications**

**1.** Procédé de synthèse acellulaire de protéines comprenant :

- la préparation d'un extrait d'embryon de plante comprenant les étapes suivantes :

(a)

(1) hacher un embryon de plante par découpage, et
(2) extraire l'embryon de plante haché à l'aide d'un solvant d'extraction,

ou
(a)' hacher un embryon de plante en présence d'un solvant d'extraction par découpage et extraire l'embryon de plante qui a été haché, dans lequel lesdites étapes de hachage et d'extraction sont réalisées en même temps, et

- l'utilisation de l'extrait d'embryon de plante pour la synthèse acellulaire de protéines.

**2.** Procédé de synthèse acellulaire de protéines selon la revendication 1, dans lequel le hachage de l'embryon de plante par découpage se fait à l'aide d'une lame tournant à grande vitesse.

**3.** Procédé de synthèse acellulaire de protéines selon l'une quelconque des revendications 1 ou 2, dans lequel l'étape de hachage de l'embryon de plante se déroule en présence d'un solvant d'extraction.

**4.** Procédé de synthèse acellulaire de protéines selon l'une quelconque des revendications 1 à 3, dans lequel le solvant d'extraction comprend au moins une substance choisie parmi le groupe comprenant : une solution tampon, des ions potassium, des ions magnésium, et un thiol antioxydant.

**5.** Procédé de synthèse acellulaire de protéines selon l'une quelconque des revendications 1 à 4, dans lequel l'embryon de plante est l'embryon du blé, de l'orge, du riz ou du maïs.

**6.** Procédé de synthèse acellulaire de protéines selon l'une quelconque des revendications 1 à 5, dans lequel l'embryon de plante est essentiellement non contaminé par un composant de l'albumen.

**7.** Solution de synthèse acellulaire de protéines **caractérisée en ce qu'**elle comprend :

- un extrait d'embryon produit par un procédé de préparation comprenant les étapes suivantes :

(a)

(1) hacher un embryon de plante par découpage, et
(2) extraire l'embryon de plante haché à l'aide d'un solvant d'extraction,

ou
(a)' hacher un embryon de plante en présence d'un solvant d'extraction par découpage et extraire l'embryon de plante qui a été haché, dans laquelle lesdites étapes de hachage et d'extraction sont réalisées en même temps,
dans laquelle l'embryon de plante est essentiellement non contaminé par un composant de l'albumen.

- et de l'ATP, GTP, de la créatine phosphate, créatine kinase, des L-aminoacides, des ions potassium et des ions magnésium.

**8.** Solution de synthèse acellulaire de protéines selon la revendication 7, dans laquelle le hachage de l'embryon de plante par découpage se fait à l'aide d'une lame tournant à grande vitesse.

**9.** Solution de synthèse acellulaire de protéines selon l'une quelconque des revendications 7 ou 8, dans laquelle l'étape de hachage de l'embryon de plante se déroule en présence d'un solvant d'extraction.

**10.** Solution de synthèse acellulaire de protéines selon l'une quelconque des revendications 7 à 9, dans laquelle le solvant d'extraction comprend au moins une substance choisie parmi le groupe comprenant : une solution tampon, des ions potassium, des ions magnésium, et un thiol antioxydant.

**11.** Solution de synthèse acellulaire de protéines selon l'une quelconque des revendications 7 à 10, dans laquelle l'embryon de plante est l'embryon du blé, de l'orge, du riz ou du maïs.

**12.** Solution de synthèse acellulaire de protéines selon l'une quelconque des revendications 7 à 11, ladite solution pour la synthèse de protéines sans cellules possédant une activité de synthèse de protéines suffisante sans que du ARNt ne soit ajouté.

**13.** Trousse pour la réalisation de la synthèse acellulaire de protéines **caractérisée en ce qu'**il comprend la solution pour la synthèse acellulaire de protéines selon l'une quelconque des revendications 7 à 12.

Fig.1

Fig.2

## Fig.3

## Fig.4

## Fig.5

**(A)**

M    RNase A
1pg   10pg   100pg  1000pg

**(B)**

The extract of the   The extract of the
present invention   conventional method
10    30    10     30   min

## Fig.6

—◇— RNase A 1pg

—□— RNase A 10pg

—▲— The extract of the present invention

—■— The extract of the conventional method

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 6098790 A
- JP 6225783 A
- JP 7000194 A
- JP 9000291 A
- JP 7147992 A

- WO 0068412 A
- WO 9307287 A
- CN 1288655 A
- WO 0127260 A


**Non-patent literature cited in the description**

- **BARNABAS et al.** *Agricultural sciences,* 1994, vol. 3, 199-205
- **DENYER et al.** *Planta,* 1995, vol. 197, 57-62
- **A.S. SPIRIN et al.** *Science,* 1988, vol. 242, 1162-1164

- **KIKAWA et al.** *The 21st meeting of Molecular Biology Society of Japan*
- **ENDO, Y et al.** *PNAS,* 18 January 2000, vol. 97 (2), 559-564
- **MADIN, K et al.** *Proc. Natl. Acad. Sci. USA,* 2000, vol. 97, 559-564